# EUROPEAN PATENT APPLICATION

(11) **EP 4 044 624 A1**
(43) Date of publication of application: **17.08.2022**
(21) Application number: 21157224.3
(22) Date of filing: 15.02.2021
(51) Int. Cl.: H04R 25/00, A61B 5/16

(54) **TRACKING HAPPY MOMENTS OF HEARING DEVICE USERS**

(71) Applicant: Sonova AG, 8712 Stäfa (CH)
(72) Inventor: Georganti, Eleftheria, 8702 Zollikon (CH); Lawrence, Melissa, 8620 Wetzikon (CH); Feilner, Manuela, 8132 Egg (CH); Lemke, Ulrike, 8053 Zürich (CH); Wessel, Thomas, 8708 Männedorf (CH)
(74) Representative: Qip Patentanwälte Dr. Kuehn & Partner mbB

(57) **Abstract**

A method for tracking happy moments of a user wearing a hearing device (12) comprising at least one microphone (20) and at least one classifier (48) configured to detect specific states of the user or of the user's environment based on a sensor signal indicative of the user's mood. The method comprises: receiving from the at least one microphone (20) or from a further sensor (50) at least one sensor signal; identifying, by the at least one classifier (48), one or more of the detected specific states of the user or of the user's environment as the happy moment, by evaluating the at least one sensor signal; recording at least the occurrence of the identified happy moment in a hearing system (10), part of which the hearing device (12) is; and, based on the recording, generating a happy moment output to notify the user of the identified happy moment and / or to stimulate an audio impression associated with the identified happy moment to evoke a further happy moment for the user.

## Description

### FIELD OF THE INVENTION

The invention relates to a method, a computer program and a computer-readable medium for tracking happy moments of a user wearing a hearing device. The hearing device comprises at least one microphone and at least one classifier which is configured to detect and classify specific states the user or the user's environment which are relevant for the user's mood. Furthermore, the invention relates to a hearing system comprising at least one hearing device of this kind and optionally a connected user device, such as a smartphone.

### BACKGROUND OF THE INVENTION

Hearing devices are generally small and complex devices. Hearing devices can include a processor, microphone, an integrated loudspeaker as a sound output device, memory, housing, and other electronical and mechanical components. Some example hearing devices are Behind-The-Ear (BTE), Receiver-In-Canal (RIC), In-The-Ear (ITE), Completely-In-Canal (CIC), and Invisible-In-The-Canal (IIC) devices. A user can prefer one of these hearing devices compared to another device based on hearing loss, aesthetic preferences, lifestyle needs, and budget.

Life in modern societies is becoming challenging for people, and many people are struggling to say that they have a happy life. Problems appear in various aspects of life, such as work, family, health or simply the fact that people are aging, and these affect people's mood and behavior, often leading to pessimistic ways of thinking or even depression. People are often overwhelmed by information, especially information about the risks that can anytime appear in their life (health problems, financial problems, political situations, climate change-related problems, environmental pollution etc.).

In the fields not related to hearing devices, quite some prior art on emotion recognition and monitoring exists. For example, in "Laughter detection for on-line human robot interaction" by M. Tahon, L. Devillers, Proceedings of the 4th Interdisciplinary Workshop on Laughter and Other Non-verbal Vocalisations in Speech, 14-15 April 2015, a method for the automatic detection of laughs in a real-time human-machine interaction is introduced. They show that the adaptation algorithm can obtain appropriate emotional prosody features, and at least several emotions can be re-synthesized. Further, in "Emotion in Speech: Recognition and Application to Call Centers" by V. A. Petrushin, 1999, a method for emotion recognition for call-centers is introduced, proposing a method that is able to detect five emotions: happiness, anger, sadness, fear, and normal (unemotional) state. Furthermore, in US 2009/0313019 A1, an emotion recognition apparatus is proposed that is able to perform accurate and stable speech-based emotion recognition, independent of language and prosodic information. It is also known that an emotional state of the user may be, for example, directly determined from an electroencephalographic (EEG) signal, see, for example, "Multiple feature fusion for automatic emotion recognition using EEG signals" by Liu et al. in IEEE, 2018.

On the other hand, in order to provide an improved way of adjusting parameters of a hearing device to the needs and preferences of an individual using the hearing device or in order to provide a way of influencing a state of mind of this individual, WO 2012/072141 A1 proposes a method comprising the steps of measuring at least one magnitude related to a state of mind of said individual, obtaining audio signals dependent on a result of this measuring, and converting them into signals to be auditorily perceived by the individual. WO 2012/072141 A1 describes a state of mind of an individual only in a very general manner, namely as comprising said individual's mood, emotions, feelings or an affect said individual has.

A detailed study on the effect of physiological factors on the perception of a happy moment is given, for instance, by Alexander et al. in "The neuroscience of positive emotions and affect: Implications for cultivating happiness and wellbeing", Neuroscience & Biobehavioral Reviews, Volume 121, 2021, Pages 220-249, ISSN 0149-7634. The effect of the user's environment on the perception of a happy moment is explored, for instance, in Helliwell et al., eds. 2020, World Happiness Report 2020, ISBN 978-1-7348080-0-1.

### DESCRIPTION OF THE INVENTION

It is an objective of the invention to provide an alternative or improved method and system employing mood state recognition in a hearing device in order to still better support a hearing device user in his everyday life.

This objective is achieved by the subject-matter of the independent claims. Further exemplary embodiments are evident from the dependent claims and the following description.

A first aspect of the invention relates to a method for tracking happy moments of a user wearing a hearing device which comprises at least one microphone and at least one classifier as described in the following.

The method may be a computer-implemented method, which may be performed automatically by a hearing system, part of which the user's hearing device is. The hearing system may, for instance, comprise one or two hearing devices used by the same user. One or both of the hearing devices may be worn on or in an ear of the user. A hearing device may be a hearing aid, which may be adapted for compensating a hearing loss of the user. Also, a cochlear implant may be a hearing device. The hearing system may optionally further comprise at least one connected user device, such as a smartphone, smartwatch, other devices carried by the user or a personal computer of the user etc.

According to an embodiment of the invention, the method comprises receiving at least one of the following signals: an audio signal from the at least one microphone or a sensor signal from at least one further sensor. The further sensor(s) may be any type(s) of physical sensor(s) - e.g. an accelerometer or an optical sensor or temperature sensor etc. - integrated in the hearing device or possibly also in a connected user device such as a smartphone or a smartwatch.

The at least one classifier is configured to evaluate thus received signals so as to detect specific states of the user and of the user's environment which are relevant for the user's mood and/or which are based on a sensor signal indicative of the user's mood. According to an embodiment of the invention, the method further comprises identifying one or more of thus detected specific states as happy moments of the user by the at least one classifier, based on evaluating the received sensor signals.

According to an embodiment of the invention, the method further comprises recording at least the occurrence of the identified happy moment (optionally along with one or more predetermined types of additional happy moment information related to it) in the hearing system. The recording may, for instance, be implemented by saving suitable data in a memory of the hearing device or a connected device.

According to an embodiment of the invention, based on the recording, the method further comprises generating a happy moment output by the hearing system at a predetermined or to-be-determined later time point. The generated happy moment output is configured to notify the user of the identified happy moment and/or to stimulate an audio impression associated with the identified happy moment to evoke a further happy moment for the user. The generated happy moment output also may be configured such as to let the user recall the identified happy moment, or such as to repeat or evoke the same or a similar happy moment for the user at said later time point. Various examples of such a happy moment output generation are described herein below. A suitable happy moment output may, for example, be in form of at least one of the following: a sound output, or an optical effect, or textual or graphical information presented to the user.

As an example of a "similar happy moment" generation, a sound once identified as "a happy moment" could be blended with similar sounds or could be emphasized, e.g. by using a higher amplification or an increased noise reduction. It could also be blended with suitable optical or vibrational effects etc.

A basic idea of the present invention is to propose a hearing system and method systematically providing to people some positive messages that are related to "happiness" in their everyday life. The idea is that these positive messages generated for the hearing device users during daily life could boost their optimistic way of thinking about life and potentially make them seek more of those moments in their lives. For this, some methods to track happiness in everyday life could be employed, as described in more detail herein below.

In other words, the idea of this invention is to introduce a framework for hearing aids that can be used to indicate whether specific moments throughout the daily lives of their users can be considered as happy. With the help of hearing devices sitting on the ear, where microphones or other sensors are present, the presented system and method can be able to: track happy moments in the people's lives; having a recording automatically made (and potentially creating a timestamp) when a happy moment is detected; and using the timestamp, or at least a record when the happy event occurred, and inform or remind the user at that moment or later.

Herein proposed is a complete solution (framework) dedicated to hearing devices that tracks happy moments of users based on a combination of sensory inputs and methods. The proposed system requires at least one hearing device and, optionally, at least one connected user device, that contain the following components: at least a microphone, potentially health-monitoring related sensors (such as heart rate monitor, EEG sensors, accelerometers etc.) or some optical sensor(s) (e.g. camera, charged coupled device, field effect transistors (FET), or detectors of multiple wavelengths) or any other types of sensors.

A first assumption for the above-described idea is that, when hearing device users are laughing, or singing, they are most probably in a happy mood. Thus, with the help of a classifier designed as a laugh or singing detector this could be tracked. In particular, as also known in the art, the speech itself can contain various indicators of an emotional state of the user, for instance pitch, intonation, spectral envelope, or prosody of the user's voice. Regarding the prosody of the own voice used to detect emotional status, many methods known in this direction use artificial intelligence (Al) techniques, but not only. In order to increase the accuracy of the detection, rhythmic motion during laughing could be verified by correlating audio information with rhythmic movement pattern, which is known to be mainly around 8 Hz. Additional sensors such as ear-EEG, could be also used for happy moment identification.

More generally, according to an embodiment of the invention, the step of identifying a happy moment of the user includes detecting one or more of the following states of the user or of his environment: a laughing of the user; a singing or whistling of the user; a predetermined sound pattern in the acoustic environment of the user (such as favorite music, voice of loved ones, wild birds singing, sea waves sound etc.); predetermined lighting conditions in the environment of the user (such as fireworks or candle light); presence of a significant other person or animal (parents, children, partner, friends, pets, animals in general); a predetermined movement of the user's body (such as dancing, jumping, playing an instrument, or joyful/cheerful moves or gestures such as applauding etc.); a predetermined heart-beat pattern of the user; individually predetermined desired environmental or weather conditions (such as rain or sunshine or snow or thunder or waterfall etc.).

Moreover, the user's movements, based on which happiness itself is detected by a suitable movement sensor, may not only include the rhythmic movement correlated with laughing or with another speech characteristics related to happiness, but also based on another rhythmical movement of the user (e.g. tapping by a foot or dancing) correlated with a music content presented to the user or a singing voice of the user, which can indicate a good mood of the user.

Therefore, in the course of identifying the happy moments, the audio signal may be received and processed by a general sound detector, which may be an own voice detector in specific cases but may also detect music in other specific cases. Such a general sound detector may include at least one of: the user's own voice detector or a detector of the user's acoustic environment.

According to an embodiment of the invention, the step of recording further includes creating and saving a digital timestamp of each identified happy moment in the hearing system. The digital timestamp may, for instance, be defined according to a predetermined digital format or standard, which is, for example, compatible with other devices of the hearing system.

An important further development of this embodiment is the logging of the happy moments in a memory of the hearing device or of a connected device in the hearing system. The logging of the identified happy moments may, for example, be implemented by recording at least their occurrences and their digital timestamps (optionally along with additional happy moment information as described herein below) over a predetermined time period. This allows to inform the user about the happy moments in his past and can be employed to evoke joyful memories which can be an incentive for the user to further engage into related activities and locations in the future.

Those correlated activities and locations may be determined by one or more of sensors such as the microphone, an accelerometer. a clock, a location sensor (e.g. GPS sensor), or any other type of sensor. To this end, the happy moments are preferably stored with additional happy moment information that allows the user to recall and identify the happy moments. This additional information can comprise, for instance, at least one of the following: a timestamp, or duration, or location information, or information about the user's activity, or information about an acoustic environment (e.g. people talking to the user), or other (sensor) information e.g. as described in the following.

Therefore, according to an embodiment of the invention, the above-mentioned step of recording further includes determining additional happy moment information (e.g. of one or more predetermined or learned types) associated with the identified happy moment, based on the at least one received signal. The determined additional happy moment information is then recorded along with the occurrence of the associated happy moment and is used in at least one of the following steps in future: in the step of identifying happy moments by the at least one classifier, in the step of generating a happy moment output for the user, or in the step of determining a later time point to generate the happy moment output for the user.

For example, the additional happy moment information recorded along with the occurrence of a happy moment may include information of one or more of the following types: a duration of the happy moment; a location of the user; information about the user's activity; information about an acoustic environment; data related to a health monitoring of the user. Based on an audio signal and/or the sensor signal received from of the at least one microphone, a further type of additional happy moment information may be at least one of the following: a voice of a specific person, or sounds occurring in nature, or a music type, or a TV program, or a festive event or party situation. Based on a signal received from a movement sensor, a further type of additional happy moment information may be at least one of the following: a specific walking style or velocity or other movement patterns of the user (e.g. related to a sports activity or movements corresponding to a rhythm of music, e.g. dancing). Based on a signal received from a biometric sensor, a further type of additional happy moment information may be at least one of the following: a physical state of the user (such as a heart rate, or blood pressure, or oxygen saturation level, or body temperature), or an eye movement pattern, or brain waves.

Based on an output of the classifier configured to identify one or more predetermined user activity values, a further type of additional happy moment information may be a user social interaction metric, which is indicative of the social interaction of the user. This user social interaction metric may be calculated from the identified user activity values, wherein the user activity values are distributed to predefined social interaction levels, and wherein the user social interaction metric is calculated as a function (e.g. a sum) of the user activity values times predefined correlation values which define their respective contribution to each of the social interaction levels. The predetermined user activity values may, for example, be simply equal to 1, so as to indicate the presence of the respective user activity. However, any other predetermined value may be suitable as well, depending on the type of user activity to be identified. Presenting the social interaction metric as an additional happy moment information to the user can have an educational effect to demonstrate a positive impact of social activities on the user's happiness when reminding the user about the happy moments during those activities.

According to an embodiment of the invention, a happy moment tagging interface is provided in the hearing system, configured such as to enable the user to indicate an occurrence of a happy moment, e. g. when he feels happy listening to a specific sound. In this embodiment, for each happy moment indicated (i.e. tagged as "happy moment") by the user via the happy moment tagging interface, the recording step is performed. Further, the at least one classifier may be accomplished so as to automatically identify this as a happy moment in future. To this end, for example, features of the indicated happy moment which are suitable for its sensor-based automatic recognition may be determined and saved in the hearing device. The happy moment tagging interface may be, for example, implemented as a button on a hearing device or an app on a connected smartphone of the user.

According to an embodiment of the invention, a happy moment recalling or repeating or evoking interface is provided in the hearing system, configured such as to enable the user to request a generation of a happy moment output configured such as to let him recall or such as to repeat or evoke one or more of the recorded happy moments. In this embodiment, a respective happy moment output is generated (additionally or solely) whenever requested by the user via the corresponding interface. Such an interface may be, for example, implemented as a button on a hearing device or an app on a connected smartphone of the user.

According to an embodiment of the invention, the identified happy moments are additionally graded according to a predetermined scale of happiness intensity levels (e.g. on a scale from 1 to 10), based on the at least one received signal, such as at least one of: a loudness of a laughing sound, or a degree of happiness determined based on the user's speech characteristics, or a duration of the moment. This may, for instance, be implemented using the correspondingly configured classifiers. In this embodiment, the respective levels are recorded as additional happy moment information along with the occurrence of the happy moment.

According to an embodiment of the invention, based on the recorded logging data and, as the case may be, also on the recorded additional happy moment information, a predetermined statistical evaluation of the recorded happy moments is performed. In this embodiment, in the step of generating a happy moment output, the happy moments are presented to the user in a predetermined statistical form depending on thus derived statistics, e.g. by selecting the most frequently or most rarely occurring type of happy moments, or presenting the happy moments to the user as compared with an earlier derived statistics etc.

According to an embodiment of the invention, based on the recorded logging data and, as the case may be, also on the recorded additional happy moment information, the happy moments are categorized in "recurring" happy moments that occurred already at the same or similar time, location, user activity, acoustic environment etc., and "new" happy moments associated with a new time, location, user activity, acoustic environment etc. In this embodiment, generating a happy moment output depends on the result of this categorization, e.g. by indicating it to the user or presenting the new or the recurring happy moments first etc. Here, for example, information about the new happy moments may be especially useful to point the user in a direction to improve his life.

The pre-determined or to-be-determined time of generating a happy moment output may, for example, be determined by one or more of the following: by a statistical definition for an unexpected future moment; during long speech pauses (e.g. during conference or other professional meeting pauses); coupled to an identified occurrence of similar specific states of the user or of the user's environment (e.g. in similar acoustic surroundings, such as silence or rain); by recurring time intervals with a predetermined periodicity.

According to an embodiment of the invention, the step of identifying a happy moment of the user employs one or more of the following: machine learning (by any suitable ML algorithm) based on the additional happy moment information recorded along with the occurrence of identified happy moments; an EEG signal correlation with a sound perceived from a specific sound source in the environment of the user.

Further aspects of the invention relate to a computer program for tracking happy moments of a user wearing a hearing device which comprises at least one microphone and at least one classifier configured to detect and classify specific states of the user or of the user's environment which are relevant for the user's mood, which program, when being executed by a processor, is adapted to carry out the steps of the method as described above and in the following as well as to a computer-readable medium, in which such a computer program is stored.

For example, the computer program may be executed in a processor of a hearing device, which hearing device, for example, may be carried by the person behind the ear. The computer-readable medium may be a memory of this hearing device. The computer program also may be executed by a processor of a connected user device, such as a smartphone or any other type of mobile device, which may be a part of the hearing system, and the computer-readable medium may be a memory of the connected user device. It also may be that some steps of the method are performed by the hearing device and other steps of the method are performed by the connected user device.

In general, a computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet, which allows downloading a program code. The computer-readable medium may be a non-transitory or transitory medium.

A further aspect of the invention relates to a hearing system comprising a hearing device worn by a hearing device user, as described hearin above and below, wherein the hearing system is adapted for performing the method described hearin above and below. The hearing system may further include, by way of example, a second hearing device worn by the same user and/or a connected user device, such as a smartphone or other mobile device or personal computer, used by the same user.

According to an embodiment of the invention, the hearing device comprises: a microphone; a processor for processing a signal from the microphone; a sound output device for outputting the processed signal to an ear of the hearing device user; a transceiver for exchanging data with the connected user device and/or with another hearing device worn by the same user; and at least one classifier configured to detect and classify specific states of the user or of the user's environment which are relevant for the user's mood, based on at least one of: an audio signal from the at least one microphone or a sensor signal from at least one further sensor.

It has to be understood that features of the method as described above and in the following may be features of the computer program, the computer-readable medium and the hearing system as described above and in the following, and vice versa.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 schematically shows a hearing system according to an embodiment of the invention.
Fig. 2 shows a flow diagram of a method according to an embodiment of the invention for tracking happy moments of a user wearing a hearing device of the hearing system of Fig. 1.
Fig. 3 shows a schematic block diagram of a framework (system and method) according to an embodiment of the invention.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

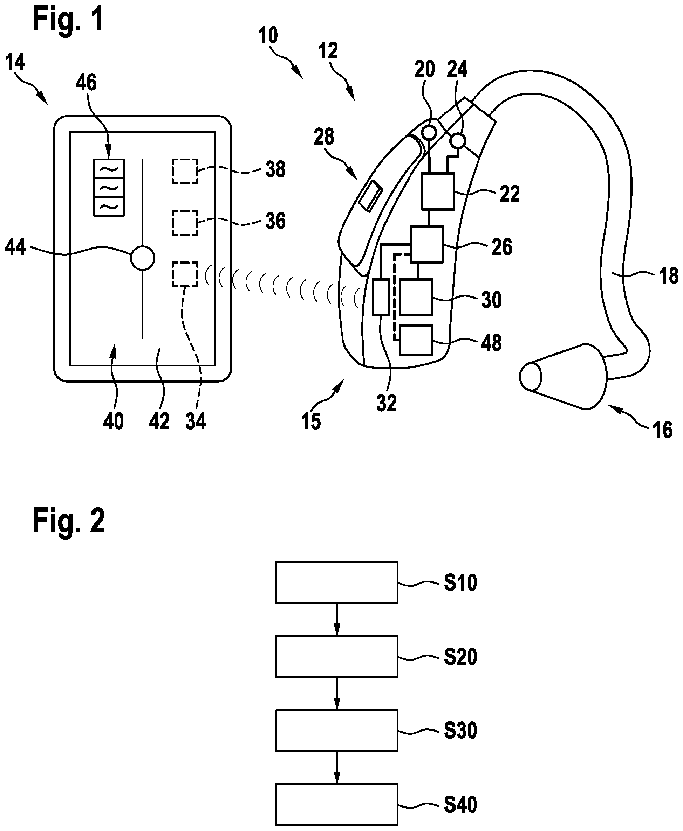

Fig. 1 schematically shows a hearing system 10 including a hearing device 12 in the form of a behind-the-ear device carried (i.e. worn) by a hearing device user (not shown) and a connected user device 14, such as a smartphone or a tablet computer or a smartwatch. It has to be noted that the hearing device 12 is a specific embodiment and that the method described herein also may be performed with other types of hearing devices, such as in-the-ear devices.

The hearing device 12 comprises a part 15 to be positioned behind the ear and a part 16 to be put in the ear channel of the user. The part 15 and the part 16 are connected by a tube 18. In the part 15, at least one microphone 20, a sound processor 22 and a sound output device 24, such as a loudspeaker, are provided. The microphone(s) 20 may receive environmental sound of the user and may generate a sound signal, the sound processor 22 may amplify the sound signal and the sound output device 24 may generate sound that is guided through the tube 18 and the in-the-ear part 16 into the ear channel of the user.

The hearing device 12 may comprise a processor 26 which is adapted for adjusting parameters of the sound processor 22 such that an output volume of the sound signal is adjusted based on an input volume. These parameters may be determined by a computer program run in the processor 26. For example, with a knob 28 of the hearing device 12, a user may select a modifier (such as bass, treble, noise suppression, dynamic volume, etc.) and levels and/or values of these modifiers may be selected, from this modifier, an adjustment command may be created and processed as described above and below. In particular, processing parameters may be determined based on the adjustment command and based on this, for example, the frequency dependent gain and the dynamic volume of the sound processor 22 may be changed. All these functions may be implemented as computer programs stored in a memory 30 of the hearing device 12, which computer programs may be executed by the processor 22.

The hearing device 12 further comprises a transceiver 32 which may be adapted for wireless data communication with a transceiver 34 of the connected user device 14, which may be a smartphone or tablet computer. It is also possible that the above-mentioned modifiers and their levels and/or values are adjusted with the connected user device 14 and/or that the adjustment command is generated with the connected user device 14. This may be performed with a computer program run in a processor 36 of the connected user device 14 and stored in a memory 38 of the connected user device 14. The computer program may provide a graphical user interface 40 on a display 42 of the connected user device 14.

For example, for adjusting the modifier, such as volume, the graphical user interface 40 may comprise a control element 44, such as a slider. When the user adjusts the slider, an adjustment command may be generated, which will change the sound processing of the hearing device 12 as described above and below. Alternatively or additionally, the user may adjust the modifier with the hearing device 12 itself, for example via the knob 28.

The user interface 40 also may comprise an indicator element 46, which, for example, displays a currently determined listening situation.

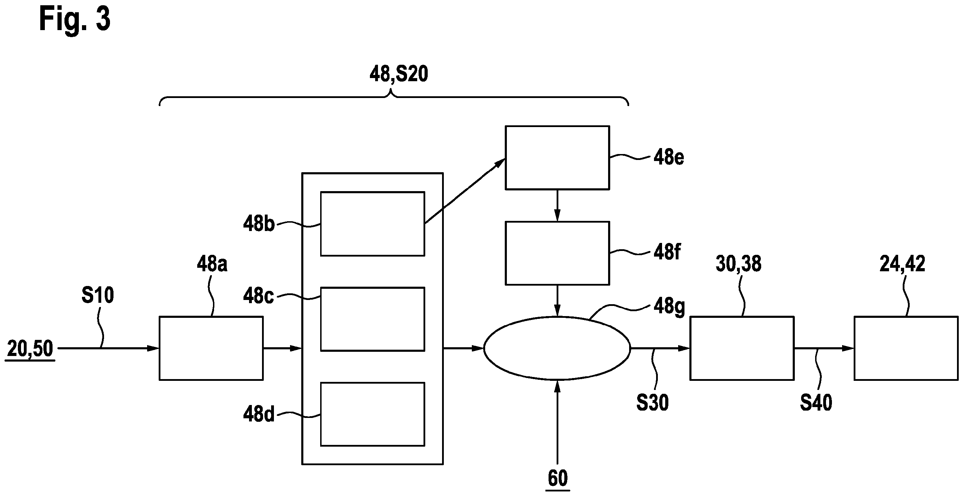

The hearing device 12 further comprises at least one classifier 48 configured to detect and classify specific states of the user and of the user's environment which are relevant for the user's mood, based on at least one of: an audio signal from the microphone(s) (20) or a sensor signal from at least one further sensor (50) (cf. Fig. 3).

The hearing system 10 shown in Fig. 1 is adapted for performing a method according to the present invention for tracking happy moments of a user wearing a hearing device 12 provided with the at least one integrated microphone 20 and the at least one classifier 48 as described in more detail herein above and below.

A happy moment, as used herein, may be any period of time during which the user experiences a mental state and/or an emotional state including pleasant and/or positive emotions which may range from a contentment to an intense joy. For instance, a happy moment may be experienced by the user as joyful, exciting, lucky, funny, humorous, sparkling, cheerful, playful, positive, thrilling, stimulating, motivating, enthusiastic, energizing, animating, vitalizing, uplifting, boosting, raising, encouraging, inspiring, connected, and/or the like. From a physiological point of view, a happy moment may be characterized as a moment during which hormones are released in the user's body causing such a mental and/or emotional state, in particular an increased level of those hormones in the user's body as compared to a base level averaged over time. Those hormones may include dopamine and/or endorphins and/or serotonin. A happy moment may also be at least partially caused by neurotransmitters transmitting a signal influencing the mental and/or emotional state between neurons. The release of the hormones and/or transmission of neurotransmitters can thus result in positive emotions of the user and can have an impact on how the user behaves and/or chooses to act.

Fig. 2 shows an example for a flow diagram of this method according to an embodiment of the invention. The method may be a computer-implemented method performed automatically in the hearing system 10 of Fig. 1.

In a first step S10 of the method, at least one of the following signals: an audio signal from the microphone(s) 20 or a sensor signal from the at least one further sensor 50 (not explicitly shown in Fig. 1) is received, e.g. by the sound processor 22 and the processor 26 of the hearing device 12.

In a second step S20 of the method, the signal(s) received in step S10 are evaluated by the one or more classifiers 48 implemented in the hearing device 12 and system 10 so as to detect specific states of the user or of the user's environment which are relevant for the user's mood and so as to identify one or more of these states as the user's happy moments. The results of this may be, for example, output by the classifier(s) 48 to the processor 26 performing the method, as only symbolically indicated by the dashed line in Fig. 1. It also may be that the classifiers 48 are implemented in the processor 26 itself or are stored as program modules in the memory so as to be performed by the processor 26. As already mentioned herein above, it also may be that all or some of the steps of the method are performed by the processor 36 of the connected user device 14 as well.

In a third step S30 of the method (referred to as the recording step herein above and below), at least the occurrence of each happy moment identified by the classifier(s) 48 is recorded in the hearing system 10, preferably along with a corresponding timestamp and optionally along with one or more predetermined types of additional happy moment information as described herein above and below. The recording may, for instance, be implemented by saving these data in the memory 30 of the hearing device 12 or in the memory 38 of the connected device 14 for further use.

In a fourth step S40, based on this recording, a happy moment output is generated such as to let the user recall the identified happy moment or such as to repeat or evoke the same or a similar happy moment for the user at a predetermined or to-be-determined later time point. Such an output may, for example, include at least one of the following: a sound output of the sound output device 24 of the hearing device 12, or an optical effect or textual or graphical information presented to the user by the connected user device 14 or by any further connected device (not shown) of the hearing system 10.

Fig. 3 schematically shows an indicative block diagram of a method according to an embodiment of the invention, which may serve as a framework for the present method. The method may be a computer-implemented method performed automatically in the hearing system 10 of Fig. 1, e.g. according to the flow diagram of Fig. 2.

As shown on the left in Fig. 3 (corresponding to step S10 of Fig. 2), the hearing device 12 captures the audio signal from its microphone(s) 20 and/or receives information from other sensors 50 provided in the hearing device 12 or elsewhere in the hearing system 10. By suitably configured classifiers 48 (48a-48g), these signal(s) are then processed in step S20 of Fig. 2 such as to extract information related to the emotional status of the hearing device user and to identify his/her "happy moments". A first assumption is that when a person is laughing, or singing, he/she is most probably in a happy mood, thus with the help of an own voice detector 48a feeding a laugh detector 48b or singing detector 48c, this could be tracked. Moreover, the prosody and other features of the user's own voice can also be used in a further emotion recognition module 48d to detect emotional status, and there are even many methods in this direction known in the art and using Al techniques, but not only. In order to increase the accuracy of the detection, rhythmic motion during laughing may be detected in a movement detector 48e and verified in a correlator 48f by correlating audio information with rhythmic movement pattern (such as e.g. mainly around 8 Hz).

As shown in Fig. 3, the outputs of all the different modules, detectors or correlators 48a-48f may be fed to the actual happiness indicator 48g configured to indicate the happy moments of the user. Additional sensors 50 such as ear-EEG and other sensors mentioned herein above and below, can also be used to feed the correlator 48f or directly the happiness indicator 48g. As mentioned with respect to Fig. 1 and Fig. 2, the output of the happiness indicator 48g stating the occurrence of each happy moment of the user (and preferably including a corresponding timestamp and optionally some additional happy moment information) is provided, for example, to the memory 30 of the hearing device 12 or to the memory 38 of the connected device 14 to be recorded there in step S30 of Fig. 2 for further use.

These records are then used in step S40 of Fig. 2 at a predetermined later time point to generate a suitable happy moment output as described herein above and below to the user, e.g. by the sound output device 24 of the hearing device 12, or by a loudspeaker, optical output device or display 42 of the connected user device 14 or any other connected device in the hearing system 10.

The movement detector 48e in Fig. 3 could also be replaced by a classifier based on a signal of a biometric sensor, for instance an EEG sensor, as further described below. Moreover, the user's movements based on which happiness itself is detected by the movement sensor may not only include the rhythmic movement correlated with laughing or with another speech characteristics related to happiness, but also based on another rhythmical movement of the user (e.g. tapping by a foot or dancing) correlated with a music content presented to the user and/or a singing voice of the user, which can indicate a good mood of the user. Thus, the invention may be described more generally by replacing the own voice detector 48a (or voice activity detector, VAD) with a general sound detector, which may be an own voice detector 48a in specific cases but may also detect music in other specific cases.

The main advantage of the idea presented in Figs. 1-3, is that the information detected and provided to the user in form of a happy moment output is positive and it is not another piece information about a potential health problem, a risk etc. Positive messages are very important according to positive psychology theory.

As a possible extension of this idea, in Fig. 3 a happy moment tagging interface 60 as described further above is provided, using which the user can indicate "happy moments" as well, and they are not only automatically tracked. For example, it can happen that a user feels happy when listening to specific sounds (e.g. sea waves, wind, snow quiet, favourite music piece, bells from the hometown, birds singing etc.). These sounds could be tagged by the user as "happy sounds", and stored in the hearing device 12 for future use. In order to introduce positive emotions, these "happy sounds" could be played back (happy moment output) at appropriate moments, e.g. later at an unexpected moment, during long speech pauses, after approval or request from the user, or in similar acoustic surroundings or could be blended with similar sounds or similar sound could be emphasized, e.g. by using a higher amplification or an increased noise reduction.

In other words, the present application describes a complete solution (framework) dedicated to hearing devices what tracks happy moments of users based on a combination of sensory inputs and methods and on their correlations.

An important embodiment is the logging of the happy moments in a memory of the hearing device 12 or of a connected device 14 in the hearing system 10. The logging of the identified happy moments may, for example, include recording at least their occurrences and their digital timestamps (optionally along with additional happy moment information as described herein below) over a predetermined time period. This may be used to inform the user about the happy moments in his past and to evoke joyful memories which can be an incentive for the user to further engage into related activities and locations in the future.

In the step of generating a happy moment output (step S40 in Fig. 2 and 3), the happy moments can be presented to the user automatically, for example, in predetermined recurring time intervals or, alternatively or additionally, after a user interaction in which the user indicates he wants to be informed about the happy moments. In particular, the happy moments can be presented in a statistical form representing e.g. a number of happy moments per time and may be compared with an earlier derived statistics. The happy moments may also be graded (e.g. on a scale from 1 to 10), for instance based on at least one of: a loudness of a laughing sound, or a degree of happiness determined based on the user's speech characteristics, or a duration of the moment. The happy moments may also be categorized in "recurring" happy moments that occurred already at a corresponding time / location / user activity / acoustic environment, and "new" happy moments associated with a new time / location / user activity / acoustic environment. Information about the new happy moments may be especially useful to point the user in a direction to improve his life.

Additional information associated with happy moments, which can be detected by a microphone, can include, for instance, at least one of the following: a voice of a specific person, sounds occurring in nature, a music type, a TV program, a festive event or party situation. In some implementations, the sound may be streamed from a remote audio source without being detected by a microphone, for instance from a music streaming provider. In some implementations, the hearing device 12 may comprise a sound classifier which can classify the ambient sound in different categories, wherein the additional information may include the category of the ambient sound in which the happy moment occurred. In some implementations, social interactions may be detected and stored as the additional information associated with a happy moment. This can have an educational effect to demonstrate a positive impact of social activities on the user's happiness when reminding the user about the happy moments during those activities.

Additional information associated with happy moments, which can be detected by a movement sensor, can include, for instance, one or more of the following: a specific walking style or velocity or other movement patterns, e.g. related to a sports activity or movements corresponding to a rhythm of music, e,g, dancing.

Additional information associated with happy moments, which can be detected by a biometric sensor, can include, for instance, at least one of: a heart rate or blood pressure or oxygen saturation level (detected, for instance, by a PPG, i.e. photoplethysmogram sensor or an ECG, i.e. electrocardiogram sensor), or a body temperature (detected by a temperature sensor), or an eye movement pattern (detected by an EOG, i.e. electrooculographic sensor), or brain waves (detected by an EEG, i.e. electroencephalographic sensor). For instance, the brain waves may indicate whether the user is sleeping or awake. The brain waves may also indicate a cognitive load of a user, which may arise, for instance, when the user has difficulty to understand a person talking to him. This may imply a reduced happiness correlated with hearing loss and an increased happiness when the hearing is improved by according settings of a hearing aid. The brain waves may also indicate an exhaustion level of the user, e.g. lack of sleep, and the happiness may be increased when the user sleeps more regularly.

More generally, the additional information may include data related to a health monitoring of the user. This can also have an educational effect to demonstrate a positive impact of healthy living on the user's happiness, e.g. regular exercising, quitting smoking, losing weight, etc. may be correlated with the user's happiness, at least on the long run. The user can then be motivated to pursue a healthy lifestyle by recalling the happy moments associated with it.

The biometric data may not only be used as additional information, but can also be used to identify a happy moment (e.g. in conjunction with the "emotion recognition" module 48d in Fig. 3). For instance, an emotional state of the user may be directly determined from an EEG signal, as known in the art, which may be employed in addition to determining the emotional state based on the user's speech characteristics. Moreover, the biometric data associated with a happy moment may be learned from other sensor data, as described herein above and below.

In some implementations, the sensor information provided from one of the sensors (e.g. microphone 20 or movement and other sensors 50), which can be employed to identify a happy moment, can also be employed to learn about the sensor information provided by another sensor typically occurring during happy moments. Any machine learning (ML) algorithm may be suitable. For instance, the ML algorithm may find that laughing (detected by a microphone 20) is correlated with a specific movement pattern of the user (detected by the movement sensor), which then can be learned. After the learning, the movement pattern can be employed to identify the happy moment independently from the sound information and/or to enhance the reliability of the happiness detection based on both sound and movement information. The user may also be informed about his movement behaviour and/or voice behaviour during happy moments.

Further, an EEG signal may already be naturally correlated with a sound perceived from a specific sound source in the environment of the user, which may, as also known in the art, be derived by comparing the EEG signal with the sound signal and thus may be employed to verify a happy state (i.e. happy moment) related to the sound source and/or to provide additional information related to the happy state which can be logged. A natural correlation between an EEG signal and a movement of the user may also be employed to verify a happy state related to the movement and/or to provide additional information related to the happy state which can be logged.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 10: hearing system
- 12: hearing device
- 14: connected user device
- 15: part behind the ear
- 16: part in the ear
- 18: tube
- 20: microphone(s)
- 22: sound processor
- 24: sound output device
- 26: processor
- 28: knob
- 30: memory
- 32: transceiver
- 34: transceiver
- 36: processor
- 38: memory
- 40: graphical user interface
- 42: display
- 44: control element, slider
- 46: indicator element
- 48: classifier
- 48a-g: different classifier types or different elements included in a classifier
- 50: further or other or additional sensors
- 60: happy moment tagging interface

## Claims

1. A method for tracking happy moments of a user wearing a hearing device (12) comprising at least one microphone (20) and at least one classifier (48) which is configured to detect and classify specific states of the user or of the user's environment based on a sensor signal indicative of the user's mood, the method comprising:
receiving from the at least one microphone (20) or from a further sensor (50) at least one sensor signal;
identifying, by the at least one classifier (48), one or more of the detected specific states of the user or of the user's environment as the happy moment, by evaluating the at least one sensor signal;
recording at least the occurrence of the identified happy moment in a hearing system (10), part of which the hearing device (12) is;
and, based on the recording, generating a happy moment output to notify the user of the identified happy moment and / or to stimulate an audio impression associated with the identified happy moment to evoke a further happy moment for the user.

2. The method of claim 1, wherein the step of recording further includes
creating and saving a digital timestamp of each identified happy moment in the hearing system (10); or
logging the identified happy moments by recording at least their occurrences and their digital timestamps over a predetermined time period.

3. The method of claim 1 or 2, wherein the step of recording further includes
determining additional happy moment information associated with the identified happy moment, based on the at least one received signal;
recording the determined additional happy moment information along with the occurrence of the associated happy moment; and
using thus recorded additional happy moment information in at least one of: the step of identifying happy moments by the at least one classifier in future, the step of generating a happy moment output for the user, or the step of determining a later time point to generate the happy moment output for the user.

4. The method of claim 3, wherein the additional happy moment information recorded along with the occurrence of a happy moment includes one or more of the following:
a duration of the happy moment;
a location of the user;
information about the user's activity;
information about an acoustic environment;
based on the sensor signal received from the at least one microphone (20), a voice of a specific person, or sounds occurring in nature, or a music type, or a TV program, or a festive event or party situation;
based on a signal received from a movement sensor, at least one of: a specific walking style or velocity or other movement patterns of the user;
based on a signal received from a biometric sensor, at least one of: a physical state of the user, or an eye movement pattern, or brain waves;
based on an output of the classifier configured to identify one or more predetermined user activity values: a user social interaction metric, which is indicative of the social interaction of the user and is calculated from the identified user activity values, wherein the user activity values are distributed to predefined social interaction levels, and wherein the user social interaction metric is calculated as a function of the user activity values times predefined correlation values which define their respective contribution to each of the social interaction levels;
data related to a health monitoring of the user.

5. The method of one of the previous claims, wherein
a happy moment tagging interface (60) is provided in the hearing system (10), configured such as to enable the user to indicate an occurrence of a happy moment; and
for each happy moment indicated by the user via the happy moment tagging interface (60), the recording step is performed and the at least one classifier (48) is accomplished so as to automatically identify it as a happy moment in future.

6. The method of one of the previous claims, wherein
a happy moment recalling or repeating or evoking interface is provided in the hearing system (10), configured such as to enable the user to request a generation of a happy moment output configured such as to let him recall or such as to repeat or evoke one or more of the recorded happy moments; and
a respective happy moment output is generated whenever requested by the user via the corresponding interface.

7. The method of one of the previous claims, wherein
the identified happy moments are additionally graded according to a predetermined scale of happiness intensity levels, based on the at least one received signal; and
the respective levels are recorded as additional happy moment information along with the occurrence of the happy moment.

8. The method of one of the claims 2 to 7, wherein
based on at least one of: the recorded logging data or the recorded additional happy moment information, a predetermined statistical evaluation of the recorded happy moments is performed; and
in the step of generating a happy moment output, the happy moments are presented to the user in a predetermined statistical form depending on thus derived statistics.

9. The method of one of the claims 2 to 8, wherein
based on at least one of: the recorded logging data or the recorded additional happy moment information, the recorded happy moments are categorized in recurring happy moments **characterized by** a similar or corresponding time or state of the user or of the user's environment, and new happy moments associated with a new time or a new specific state of the user or of the user's environment; and
generating a happy moment output depending on the result of this categorization.

10. The method of one of the previous claims, wherein the time of generating a happy moment output is determined by one or more of the following:
by a statistical definition for an unexpected future moment;
during long speech pauses;
coupled to an identified occurrence of similar specific states of the user or of the user's environment;
by recurring time intervals with a predetermined periodicity.

11. The method of one of the previous claims, wherein the step of identifying a happy moment of the user includes detecting one or more of the following states of the user or of his environment:
a laughing of the user;
a singing or whistling of the user;
a predetermined sound pattern in the acoustic environment of the user;
predetermined lighting conditions in the environment of the user;
presence of a significant other person or animal;
a predetermined movement of the user's body;
a predetermined heart-beat pattern of the user;
individually predetermined desired environmental or weather conditions.

12. The method of one of the previous claims, wherein the step of identifying a happy moment of the user employs one or more of the following:
machine learning based on the additional happy moment information recorded along with the occurrence of identified happy moments;
an EEG signal correlation with a sound perceived from a specific sound source in the environment of the user.

13. A computer program for tracking happy moments of a user wearing a hearing device (12) comprising at least one microphone (20) and at least one classifier (48) which is configured to detect and classify specific states of the user or of the user's environment which are relevant for the user's mood, which program, when being executed by a processor (26, 36), is adapted to carry out the steps of the method of one of the previous claims.

14. A computer-readable medium, in which a computer program according to claim 13 is stored.

15. A hearing system (10) comprising a hearing device (12) worn by a hearing device user and a connected user device (14), wherein the hearing device (12) comprises:
a microphone (20);
a processor (26) for processing a signal from the microphone (20);
a sound output device (24) for outputting the processed signal to an ear of the hearing device user;
a transceiver (32) for exchanging data with the connected user device (14);
at least one classifier (48) configured to detect and classify specific states of the user or of the user's environment which are relevant for the user's mood, based on at least one of: an audio signal from the at least one microphone (20) or a sensor signal from at least one further sensor (50); and
wherein the hearing system (10) is adapted for performing the method of one of claims 1 to 12.
